# EUROPEAN PATENT APPLICATION

(11) **EP 4 260 848 A1**
(43) Date of publication of application: **18.10.2023**
(21) Application number: 22167546.5
(22) Date of filing: 11.04.2022
(51) Int. Cl.: A61K 9/20, A61K 31/506

(54) **PHARMACEUTICAL COMPOSITION FOR SOLID DOSAGE FORM CONTAINING NILOTINIB AND PROCESS FOR ITS PREPARATION**

(71) Applicant: Lotus Pharmaceutical Co., Ltd., 11046 Taipei City (TW)
(72) Inventor: Chen, Hongze, 54066 Nantou City (TW); Gattani, Yogesh Sevaramji, 54066 Nantou City (TW); Gupta, Vijender, 54066 Nantou City (TW); Chawla, Manish, 54066 Nantou City (TW)
(74) Representative: Harber IP s.r.o.

(57) **Abstract**

The invention provides a pharmaceutical composition comprising Nilotinib or a pharmaceutically acceptable salt thereof, and further comprising an insoluble polymeric diluent and an insoluble non-polymeric diluent.

The composition may be produced by a process which is manageable, controllable, simple and economical. The composition and a solid dosage form containing the composition is stable and show a good rate and degree of release of the active substance.

## Description

### TECHNICAL FIELD

The invention relates to a pharmaceutical composition of solid dosage form containing nilotinib, which is used in pharmaceuticals and medicine for the treatment of chronic myeloid leukemia and gastrointestinal stromal tumors, and to a process for its preparation.

### BACKGROUND ART

Nilotinib is an aminopyrimidine-derivative; Bcr-Abl tyrosine kinase inhibitor with antineoplastic activity. Chemically, Nilotinib is 4-methyl-N-[3-(4-methylimidazol-1-yl)-5-(trifluoromethyl)phenyl]-3-[(4-pyridin-3-ylpyrimidin-2-yl)amino]benzamide with a molecular formula C₂₈H₂₂F₃N₇O and molecular weight 529 g/mol. The structural formula of Nilotinib is:

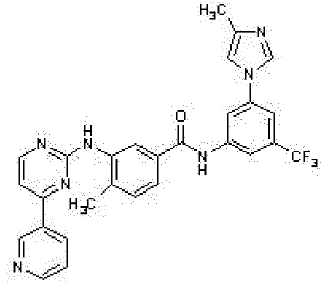

The solubility of Nilotinib in aqueous solutions decreases with increasing pH. Nilotinib is not optically active. The pKₐ₁ was determined to be 2.1; pKₐ₂ is estimated to be 5.4.

Nilotinib is an inhibitor of the protein tyrosine kinase (TK) activity of Bcr-Abl. Examples of conditions that may be treated by such therapeutic compounds include chronic myeloid leukemia and gastrointestinal stromal tumors.

Nilotinib hydrochloride monohydrate is sold under the brand name Tasigna^{®} marketed worldwide by Novartis. Nilotinib is indicated for the treatment of newly diagnosed Philadelphia chromosome positive chronic myeloid leukemia (Ph+ CML) in chronic phase and accelerated phase by inhibition of tyrosine kinase (TK) activity of Bcr-Abl in adult patients. Patent application WO 04/005281 discloses Nilotinib (4-methyl-N-[3-(4-methylimidazol-1-yl)-5-(trifluoromethyl)phenyl]-3-[(4-pyridin-3-ylpyrimidin-2-yl)amino]benzamide); the process for its manufacture and its use for treatment of disease which responds to an inhibition of protein kinase activity, especially a neoplastic disease, in particular leukemia.

Patent application WO 07/015871 discloses salts of 4-methyl-N-[3-(4-methyl-imidazol-1-yl)-5-trifluoromethyl-phenyl]-3-(4- pyridin-3-yl-pyrimidin-2-ylamino)-benzamide; and processes of preparing the same.

Patent application WO 07/015870 discloses different polymorphs such as pure crystalline Form A, B, C and D of Nilotinib, Nilotinib hydrochloride and substantially pure amorphous form of Nilotinib hydrochloride.

Nilotinib hydrochloride, which is the commercially available salt used in marketed products can exist in a number of different solid forms including anhydrates, hydrates and solvates, all of which exhibit poor aqueous solubility.

Patent application WO 08/037716 discloses pharmaceutical compositions of Nilotinib prepared by wet granulation process. It further describes problems associated with dissolution of Nilotinib compositions comprising hydrophobic inactive agents such as lubricants and provides a solution by inclusion of a surfactant during the wet granulation which enables reducing the concentration of the lubricant(s) to less than 1 % by weight of the pharmaceutical composition and achieving better solubility and dissolution.

Lubricants are essential for maintaining powder and/or granule flowability during compaction. Inadequate amount of lubricant can lead to low flowability. Low flowability of the powder may cause technological problems; major problems include uneven filling of the tablet press, substantial differences in the tablet mass and hardness of the tablets.

Furthermore, a wet granulation process requires the drug to be exposed to solvents, which increases the risk of polymorphic or crystalline change of the drug and/or its chemical degradation. Yet furthermore, incompatibilities between formulation ingredients are aggravated by the granulating solvent which tends to bring them into close contact. Additionally, the cost of wet granulation is high because of the time, labor, energy, equipment, and space required for the process.

Patent application WO 12/164578 discloses process for preparing dry-granulated pharmaceutical composition of Nilotinib comprising compacting Nilotinib hydrochloride, a water-soluble polymer selected from polyethylene glycol, polyvinyl pyrrolidone, polyethylene oxides, alkyl cellulose, hydroxypropyl methyl cellulose, hydroxypropyl cellulose or mixtures thereof, and one or more excipients selected from a diluent, a binder, a disintegrant, a surfactant, a glidant and a lubricant.

It is an object of the invention to provide a pharmaceutical composition for a solid dosage form containing Nilotinib with good pharmaceutical and biological availability, as well as a process of its preparation which is easily manageable, controllable, simple and economical. The solid dosage form should be stable and show a good rate and degree of release of the active substance.

### DISCLOSURE OF THE INVENTION

To solve the above listed technical problems, the present invention provides a pharmaceutical composition comprising Nilotinib, and further comprising a polymeric diluent and a non-polymeric diluent, both diluents being insoluble. This composition enables to achieve adequate dissolution of Nilotinib dosage forms independent of the amount of lubricant present in the dosage form, wherein the total content of lubricant(s) is more than 1 % by weight of the pharmaceutical composition, thus allowing to effectively overcome the problems associated with the low amount of lubricant(s) which maintaining excellent dissolution properties and avoiding the use of surfactant.

The term "Nilotinib" as used herein includes Nilotinib in the form of free base, a pharmaceutically acceptable salt thereof, amorphous Nilotinib, crystalline Nilotinib or any Nilotinib isomer, Nilotinib derivative, Nilotinib hydrate, Nilotinib dihydrate, Nilotinib solvate, Nilotinib prodrug and combinations thereof. Amorphous as well as crystalline forms are included.

"Salts" or "pharmaceutically acceptable salt(s)", as used herein, include inorganic or organic salts of Nilotinib, as well as hydrates, dihydrates, and solvates of the organic or inorganic salts of Nilotinib. Amorphous as well as crystalline forms are included. In an embodiment of the present invention, the salt of Nilotinib refers to hydrochloride salt, Nilotinib hydrochloride hydrate, Nilotinib hydrochloride dihydrate, Nilotinib hydrochloride anhydrous form, and combinations thereof.

The term "composition" or "pharmaceutical composition" as used herein refers to a mixture of at least one active ingredient and pharmaceutically acceptable excipients. The active ingredient is usually Nilotinib. The composition is typically a solid composition.

The term "solid dosage form" includes solid dosage forms such as tablets, capsules, granules, mini-tablets, pills, beads and the like, meant for oral administration.

As used herein, the term "excipient" refers to a pharmaceutically acceptable ingredient that is commonly used in the pharmaceutical technology for preparing pharmaceutical compositions and/or solid oral dosage formulations. Examples of categories of excipients include binders, disintegrants, lubricants, stabilizers, and diluents.

The present invention thus provides pharmaceutical compositions comprising Nilotinib, and further comprising a combination of an insoluble polymeric diluent and an insoluble non-polymeric diluent which may be an inorganic salt.

In some embodiments, the total amount of the diluents in the pharmaceutical composition is within the range from about 15 wt.% to about 65 wt.% per dosage unit, preferably from about 30 wt.% to about 40 wt.%

In some embodiments, the amount of the insoluble polymeric diluent in the pharmaceutical composition is from about 10 wt.% to about 40 wt.%, preferably from about 15 wt.% to about 32 wt.%.

In some embodiments, the amount of the insoluble non-polymeric diluent is from about 2 wt.% to about 20 wt.% per dosage unit.

The term "insoluble diluent" refers to water-insoluble diluents.

Insoluble polymeric diluents include starches, including maize starch, potato starch, rice starch, wheat starch, pregelatinized starches and Starch 1500, Starch 1500 LM grade (low moisture content grade) from Colorcon; and celluloses including microcrystalline cellulose, silicified microcrystalline cellulose (SMCC), crystalline celluloses and powdered celluloses; and mixtures thereof. Examples of crystalline celluloses include CEOLUS^{™} KG801, Avicel^{™} PH101 , PH102, PH301 , PH302 and PH-F20. Examples of microcrystalline cellulose ("MCC") include microcrystalline cellulose 114, and microcrystalline cellulose 112.

Preferably, the insoluble polymeric diluent is selected from microcrystalline cellulose, silicified microcrystalline cellulose, crystalline celluloses and powdered celluloses, and mixtures thereof.

In a particularly preferred embodiment, the insoluble polymeric diluent is silicified microcrystalline cellulose.

Insoluble non-polymeric diluents include calcium carbonate, magnesium carbonate, magnesium aluminometasilicate, dibasic calcium phosphate, directly compressible grades of dibasic calcium phosphate (Emcompress^{™}), tribasic calcium phosphate, and mixtures thereof.

Particularly preferred insoluble non-polymeric diluent is magnesium aluminometasilicate, dibasic calcium phosphate anhydrous, or a mixture thereof.

In a preferred embodiment of present invention, the insoluble non-polymeric diluent and the insoluble polymeric diluent are present in the ratio of 1:1 to 1:15, in a more preferred embodiment, the diluents are present in the ratio of about 1:1, about 1:2, about 1:3, about 1:4, about 1:5, about 1:6, about 1:7, about 1:8, about 1:9, about 1:10, about 1:11, about 1:12, about 1:13, about 1:14, about 1:15 or any fractions in between. In a more preferred embodiment, the diluents are present in ratio of 1:1 to 1:2, or 1:2 to 1:4, or 1:11 to 1:12.

The pharmaceutical composition may further comprise at least one disintegrant, at least one binder, and at least one lubricant.

Disintegrant is preferably selected from alginic acid, carboxymethylcellulose calcium, carboxymethyl cellulose, carboxymethylcellulose sodium, cross-linked sodium carboxymethylcellulose, low substituted hydroxypropyl cellulose, colloidal silicon dioxide, croscarmellose sodium, crospovidone, guar gum, magnesium aluminium silicate, microcrystalline cellulose, methyl cellulose, polyvinylpyrrolidone, cross-linked polyvinylpyrrolidones, polacrifin potassium, starch, pregelatinized starch, sodium alginate, sodium lauryl sulphate, sodium starch glycolate, crystalline cellulose, hydroxypropyl starch and combinations thereof. A preferred disintegrant is crospovidone. The disintegrant is preferably used in an amount of from about 3% to about 30% by weight, preferably 3 to 10 % by weight, relative to the total weight of the composition.

Binder is preferably selected from acacia, guar gum, alginic acid, carbomer, dextrin, maltodextrin, methylcellulose, ethyl cellulose, hydroxyethyl cellulose, hydroxypropyl cellulose, hydroxypropyl methylcellulose, carboxymethylcellulose sodium, magnesium aluminum silicate, polymethacrylates, povidones, copovidones, gelatin, starch, and combinations thereof. The preferred binder is hydroxypropyl methylcellulose. The binder, if used, is preferably used in an amount of from about 2% to about 7% by weight, relative to the total weight of the composition.

Lubricant is preferably selected from magnesium stearate, zinc stearate, calcium stearate, stearic acid, colloidal silicon dioxide, glyceryl palmitostearate, vegetable oils, polyethylene glycols, polyvinyl alcohols, talc, sodium benzoate, sodium stearyl fumarate, magnesium oxide, poloxamer, sodium lauryl sulphate, polyoxyethylene monostearate, cocoa butter, hydrogenated vegetable oils, mineral oil, polysaccharides, and combinations thereof. The preferred lubricant is sodium stearyl fumarate. The lubricant is preferably used in an amount of at least 1% by weight, in a more preferred embodiment the amount used is at least 1.4% by weight, or 1.5 to 2% by weight, relative to the total weight of the composition.

The pharmaceutical composition of the present invention does not contain surfactants; it is surfactant-free.

The amount of Nilotinib or a pharmaceutically acceptable salt thereof is 20 to 70% by weight, preferably 50 to 60% by weight, relative to the total weight of the composition.

All amounts in % are meant to be % by weight, relative to the total weight of the composition, unless indicated otherwise. The terms "%", "wt.%" and "% by weight" are synonymous.

The pharmaceutical composition of the present invention is prepared by dry granulation or by wet granulation. In a preferred embodiment, the present composition is prepared by dry granulation.

In an embodiment of the present invention dry granulation is the preferred process for manufacturing granules to be filled in the capsules. In a further embodiment of the present invention the dry granulation can be carried out as a single step compaction or multicycle compaction process. In a preferred embodiment the dry granulation is carried out in multicycle compaction process.

The pharmaceutical composition of the present invention may be formulated into granular composition and filled into capsules. The formulation may contain an intragranular part and an extragranular part. The intragranular part typically contains the active ingredient Nilotinib, the binder (if present), part (preferably about half) of the total amount of the lubricant, and part or all of the total amount of the diluents. The extragranular part typically contains the disintegrant, part (preferably about half) of the total amount of the lubricant, and optionally part of the total amount of the diluents.

The pharmaceutical composition of the present invention preferably comprises Nilotinib, dibasic calcium phosphate and/or magnesium aluminometasilicate, silicified microcrystalline cellulose, sodium stearyl fumarate, crospovidone, and hydroxypropyl methyl cellulose. The components may preferably be in the amounts and ratios described herein above. Nilotinib may be in the form of Nilotinib hydrochloride.

### BRIEF DESCRIPTION OF THE FIGURES

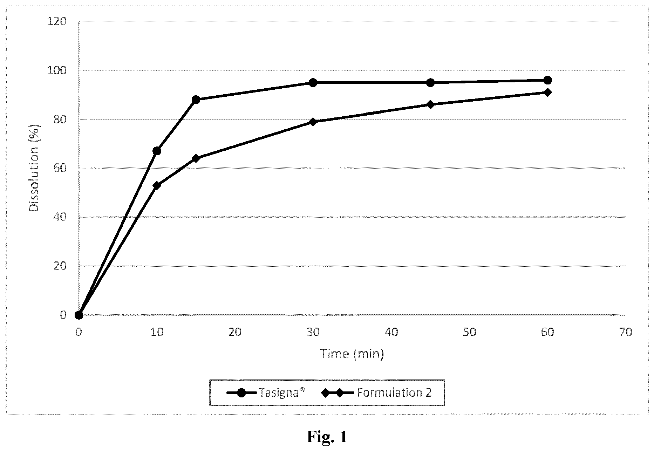

Figure 1: Comparative dissolution of Tasigna^{®} 200 mg with Formulation 2.

### EXAMPLES

The examples of the present invention are provided to illustrate the present invention in more detail. However, the examples are in no way intended to be limiting the scope of the present invention which is determined by the wording of the claims.

### Example 1. Preparation of Nilotinib Capsules by dry granulation - roller compaction

**Table 1:**

| **Ingredients** | **Function** | **Formulation 1** | **Formulation 2** |
|---|---|---|---|
| **Intragranular part** | | | |
| Nilotinib HCl | API | 227.4 | 227.4 |
| Dibasic calcium phosphate anhydrous | diluent | 60.0 | 10.0 |
| HPMC | binder | 20.0 | 20.0 |
| Sodium Stearyl fumarate | lubricant | 3.0 | 3.0 |
| Poloxamer 188 | surfactant | - | - |

| **Extra granular part** | | | |
|---|---|---|---|
| SMCC HD90 | diluent | 70.6 | 120.6 |
| Crospovidone XL | disintegrant | 16.0 | 16.0 |
| Sodium Stearyl fumarate | lubricant | 3.0 | 3.0 |
| **Total Weight** | | 400 | 400 |

### Manufacturing process:

1) Weighed accurate quantity of Nilotinib hydrochloride dihydrate and passed through the co-mill.
2) Weighed and sifted dibasic calcium phosphate anhydrous, Hydroxypropyl methylcellulose (HPMC) E15a.
3) Blend the material of step 1 and step 2 in a blender.
4) Lubricate the blend using pre-dispensed and sifted Sodium stearyl fumarate; and mix in a blender.
5) Compact the material of step 4 using roller compactor.
6) Milled the compacted material of step 5 using mesh 0.8mm.
7) Blend the material of step 6 along with pre-dispensed and sifted Silicified microcrystalline cellulose HD 90 and crospovidone, and mix in a blender.
8) Lubricate the blend using pre-dispensed and sifted Sodium stearyl fumarate, and mix in a blender.
9) Capsule filing: Fill the above lubricated blend using hard gelatin capsule shell.

**Table 2: Bioequivalence study data comparison with Tasigna^{®}**

| **Parameter** | **RLD** | **Formulation 1** | **Ratio** |
|---|---|---|---|
| **Cmax** | 565.69 | 681.655 | 120.50 |
| **AUC** | 13666.19 | 14382.621 | 105.24 |

The mean ratio of Cmax and AUC falls within 80.00 -125.00 range according to the GUIDELINE ON THE INVESTIGATION OF BIOEQUIVALENCE, Doc. Ref.: CPMP/EWP/QWP/1401/98 Rev. 1/ Corr. The test was performed as described in this document.

### Example 2. Preparation of Nilotinib capsules by dry granulation multi cycle compaction

**Table 3:**

| **Ingredients** | **Formulation 3** | **Formulation 4** | **Formulation 5** |
|---|---|---|---|
| **Intra granular** | | | |
| Nilotinib HCl | 227.4 | 227.4 | 227.4 |
| Sodium Stearyl fumarate | 3.0 | 3.0 | 3.0 |
| Poloxamer 188 | - | - | - |
| HPMC | - | - | 20.0 |
| Magnesium Aluminometasilicate, | -- | 60.0 | - |
| Dibasic calcium phosphate anhydrous | -- | - | 60.0 |
| SMCC HD90 | - | 90.6 | - |

| **Extra-Granular** | | | |
|---|---|---|---|
| SMCC HD90 | 90.6 | - | 70.6 |
| Dibasic calcium phosphate anhydrous | 60.0 | - | - |
| Crospovidone XL | 16.0 | 16.0 | 16.0 |
| Sodium Stearyl fumarate | 3.0 | 3.0 | 3.0 |
| Total Weight | 400 | 400 | 400 |

### Manufacturing process:

1. Blend in shifted Nilotinib, and the in-actives in the intragranular part; in a blender.
2. Lubricate the blend using Sodium stearyl fumarate and mix in a blender.
3. Compact the material of step 2 using roller compactor.
4. Mill the compacted material of step 3 and re-compact by using roller compactor.
5. Blend the material of step 4 along with excipients in the extra granular part; and mix in a blender
6. Capsule filing: Fill the above lubricated blend using hard gelatine capsule shell.

**Table 4: Bioequivalence study data comparison with Tasigna^{®}**

| **Parameter** | **Tasigna** | **Formulation 5** | **Ratio** |
|---|---|---|---|
| **Cmax** | 490.760 | 546.999 | 111.46 |
| **AUC** | 8989.400 | 8915.659 | 99.18 |

The mean ratio of Cmax and AUC falls within 80.00 -125.00 range according to the GUIDELINE ON THE INVESTIGATION OF BIOEQUIVALENCE, Doc. Ref.: CPMP/EWP/QWP/1401/98 Rev. 1/ Corr. The test was performed as described in this document.

**Table 5: 6 months stability at 40°C and 75% RH of Formulation 5:**

| **Nilotinib Capsules 200mg** | | |
|---|---|---|
| Sample information | Initial | 6M - 40°C/75%RH |
| Water content (%) | 5.6 | 5.2 |
| FP - Assay (%) | 99.1 | 104.3 |
| FP - Related Substance | | |
| Total impurities (%) | 0.1% | 0.1% |

| Genotoxic impurity - Aniline derivative | | |
|---|---|---|
| Aniline derivative (ppm) | 2 | 4.5 |
| FP - Dissolution (%) 1000mL, Paddle_100rpm_Helix sinker | Mean= 100% | Mean= 100% |
| | RSD= 0.7% | RSD= 7.9% |
| XRD (Retain the polymorphic form on stability) | Complies | Complies |

| | | |
|---|---|---|
| N.R.: Not Reported FP: Final product | | |

## Claims

1. A pharmaceutical composition comprising Nilotinib or a pharmaceutically acceptable salt thereof, and further comprising an insoluble polymeric diluent and an insoluble non-polymeric diluent.

2. The pharmaceutical composition of claim 1, wherein the insoluble polymeric diluent is selected from microcrystalline cellulose, silicified microcrystalline cellulose, crystalline cellulose, powdered cellulose, and mixtures thereof; preferably silicified microcrystalline cellulose.

3. The pharmaceutical composition of claim 1 or 2, wherein the insoluble non-polymeric diluent is selected from calcium carbonate, magnesium carbonate, magnesium aluminometasilicate, dibasic calcium phosphate, tribasic calcium phosphate, and mixtures thereof; preferably magnesium aluminometasilicate and/or dibasic calcium phosphate.

4. The pharmaceutical composition of any one of claims 1 to 3, wherein the total amount of the diluents in the pharmaceutical composition is within the range from about 15 wt.% to about 65 wt.% per dosage unit, preferably from about 30 wt.% to about 40 wt.%.

5. The pharmaceutical composition of any one of claims 1 to 4, wherein the amount of the insoluble polymeric diluent in the pharmaceutical composition is from about 10 wt.% to about 40 wt.%, preferably from about 15 wt.% to about 32 wt.%; and the amount of the insoluble non-polymeric diluent is from about 2 wt.% to about 20 wt.% per dosage unit.

6. The pharmaceutical composition of any one of claims 1 to 5, further comprising a disintegrant selected from alginic acid, carboxymethylcellulose calcium, carboxymethyl cellulose, carboxymethylcellulose sodium, cross-linked sodium carboxymethylcellulose, low substituted hydroxypropyl cellulose, colloidal silicon dioxide, croscarmellose sodium, crospovidone, guar gum, magnesium aluminium silicate, microcrystalline cellulose, methyl cellulose, polyvinylpyrrolidone, cross-linked polyvinylpyrrolidones, polacrifin potassium, starch, pregelatinized starch, sodium alginate, sodium lauryl sulphate, sodium starch glycolate, crystalline cellulose, hydroxypropyl starch and combinations thereof; preferably the disintegrant is crospovidone.

7. The pharmaceutical composition of any one of claims 1 to 6, further comprising a binder selected from acacia, guar gum, alginic acid, carbomer, dextrin, maltodextrin, methylcellulose, ethyl cellulose, hydroxyethyl cellulose, hydroxypropyl cellulose, hydroxypropyl methylcellulose, carboxymethylcellulose sodium, magnesium aluminum silicate, polymethacrylates, povidones, copovidones, gelatin, starch, and combinations thereof; preferably the binder is hydroxypropyl methylcellulose.

8. The pharmaceutical composition of any one of claims 1 to 7, further comprising more than 1 wt. % of lubricant selected from magnesium stearate, zinc stearate, calcium stearate, stearic acid, colloidal silicon dioxide, glyceryl palmitostearate, vegetable oils, polyethylene glycols, polyvinyl alcohols, talc, sodium benzoate, sodium stearyl fumarate, magnesium oxide, poloxamer, sodium lauryl sulphate, polyoxyethylene monostearate, cocoa butter, hydrogenated vegetable oils, mineral oil, polysaccharides, and combinations thereof; preferably the lubricant is sodium stearyl fumarate.

9. The pharmaceutical composition of any one of claims 1 to 8, comprising:
- 20 wt.% to 70 wt.%, preferably 50 wt.% to 60 wt.%, of Nilotinib or a pharmaceutically acceptable salt thereof;
- about 15 wt.% to about 65 wt.% per dosage unit, preferably from about 30 wt.% to about 40 wt.%, of diluents;
- more than 1 wt.% of lubricant;
- about 3 wt.% to about 30 wt.%, preferably 3 wt.% to 10 wt.%, of at least one disintegrant;
- optionally about 2 wt.% to about 7 wt.% of at least one binder.

10. The pharmaceutical composition of any one of claims 1 to 9, which comprises Nilotinib hydrochloride, dibasic calcium phosphate and/or magnesium aluminometasilicate, silicified microcrystalline cellulose, sodium stearyl fumarate, crospovidone, and hydroxypropyl methyl cellulose.

11. A method for preparing the pharmaceutical composition according to any one of claims 1 to 10, which comprises a step of dry granulation.
